# EUROPÄISCHE PATENTANMELDUNG

(11) **EP 1 522 253 A1**
(43) Veröffentlichungstag der Anmeldung: **13.04.2005**
(21) Anmeldenummer: 04015787.7
(22) Anmeldetag: 05.07.2004
(51) Int. Cl.: A61B 1/06

(54) **Elektronisches Endoskop**

(30) Priorität: 07.10.2003 DE 10346598
(71) Anmelder: Henke-Sass, Wolf GmbH, D-78532 Tuttlingen (DE)
(72) Erfinder: Schulz, Dieter, 78570 Mühlheim a.D. (DE); Kiehn, Ralf, 78606 Seitingen-Oberflacht (DE)
(74) Vertreter: Grimm, Christian

(57) **Zusammenfassung**

Es wird bereitgestellt ein elektronisches Endoskop mit einem Schaft (1), einer Bildaufnahmeeinheit (5), die ein Objektiv (6) sowie einen diesem nachgeordneten Bildsensor (7) zur Aufnahme eines Objekts aufweist und die am distalen Ende des Schafts (1) fest mit diesem verbunden ist, einem Handgriff (4), der mit dem proximalen Ende des Schafts (1) verbunden ist, sowie mit einer mit dem Schaft (1) verbundenen Beleuchtungseinrichtung (11, 12, 13,14) zum Beleuchten des aufzunehmenden Objektes, wobei die Beleuchtungseinrichtung (11, 12, 13,14) zumindest eine Leuchtdiode (11, 12, 13, 14) aufweist.

## Beschreibung

Die Erfindung bezieht sich auf ein elektronisches Endoskop mit einem Schaft, einer Bildaufnahmeeinheit, die ein Objektiv sowie einen diesem nachgeordneten Bildsensor zur Aufnahme eines Objektes aufweist und die am distalen Ende des Schafts fest mit diesem verbunden ist, einem Handgriff, der mit dem proximalen Ende des Schafts verbunden ist, sowie mit einer mit dem Schaft verbunden Beleuchtungseinrichtung zum Beleuchten des aufzunehmenden Objekts.

Ein solches elektronisches Endoskop, das beispielsweise in der DE 196 47 855 A1 beschrieben ist, wird insbesondere im medizinischen und technischen Bereich eingesetzt. Die Beleuchtungseinrichtung umfaßt häufig eine Kaltlichtquelle, deren Licht am Handgriff in einen Lichtleiter eingekoppelt wird und vom Lichtleiter bis zum distalen Ende des Schafts übertragen wird. Dies führt zu einer aufwendigen und damit auch teueren Beleuchtungseinrichtung.

Ausgehend hiervon ist es Aufgabe der Erfindung, das elektronische Endoskop der eingangs genannten Art dahingehend weiterzubilden, daß es einfach und kostengünstig herstellbar ist.

Erfindungsgemäß wird die Aufgabe bei dem eingangs beschriebenen elektronischen Endoskop dadurch gelöst, daß die Beleuchtungseinrichtung als Lichtquelle zumindest eine Leuchtdiode aufweist. Leuchtdioden sind heutzutage Massenprodukte, die äußert kostengünstig sind und dabei auch noch eine sehr hohe Lebensdauer aufweisen. Damit ist ein einfacher Aufbau bei dem erfindungsgemäßen elektronischen Endoskop möglich, der zudem eine äußerst kostengünstige Beleuchtungsvorrichtung umfaßt.

In einer bevorzugten Weiterbildung des erfindungsgemäßen Endoskops weist dieses, insbesondere im Handgriff, eine Steuereinheit auf, mit der die zumindest eine Leuchtdiode gepulst betreibbar ist. Dies führt zu dem Vorteil, daß dadurch die Lebensdauer der Leuchtdiode weiter erhöht werden kann und dennoch genügend Licht zur Beleuchtung des aufzunehmenden Objekts von der zumindest einen Leuchtdiode zur Verfügung gestellt wird. Ferner kann beim gepulsten Betrieb die Leuchtdiode mit höherem Strom betrieben werden, wodurch vorteilhaft eine höhere Lichtstärke erreicht werden kann.

Insbesondere kann bei dem erfindungsgemäßen Endoskop die Steuereinheit derart ausgebildet sein, daß der gepulste Betrieb der zumindest einen Leuchtdiode mit der Bildaufnahmerate des Bildsensors synchronisiert ist. Dadurch wird vorteilhaft erreicht, daß das zu beleuchtende Objekt immer nur in den Zeitabschnitten beleuchtet wird, in denen es mittels des Bildsensors aufgenommen wird. Somit wird eine ausgezeichnete Beleuchtung bei gleichzeitiger Minimierung des Energiebedarfs für die zumindest eine Leuchtdiode erreicht.

In einer weiteren Ausgestaltung des erfindungsgemäßen Endoskops ist eine Stromversorgungseinheit für die zumindest eine Leuchtdiode und die Bildaufnahmeeinheit im Endoskop, insbesondere im Handgriff integriert. Dies führt zu einem sehr handlichen Endoskop, bei dem die durch die Stromkabel bei herkömmlichen Endoskopen bedingten Schwierigkeiten bei der Handhabung nicht mehr auftreten. Die Stromversorgungseinheit kann vollkommen im Endoskop integriert und nicht ausbaubar oder auch auswechselbar mit dem Endoskop verbunden sein. Insbesondere kann die Stromversorgungseinheit eine Batterie, einen Akkumulator oder eine Brennstoffzelle aufweisen. Auch ist es möglich, daß die Stromversorgungseinheit einen induktiv aufladbaren Akkumulator enthält.

Besonders bevorzugt ist es, wenn das erfindungsgemäße Endoskop insgesamt oder auch nur der Schaft derart hermetisch abgedichtet ist, daß das Endoskop bzw. der Schaff autoklavierbar ist. Unter autoklavierbar wird hier verstanden, daß das Endoskop bzw. der Schaft mindestens mehrere Minuten gesättigtem Wasserdampf von mindestens 130°C zur Sterilisation ausgesetzt werden kann, ohne daß dabei das Endoskop beschädigt wird (ohne daß insbesondere Wasserdampf in das Innere des Endoskops eindringen kann). In diesem Fall ist das Endoskop bei medizinischen Anwendungen sehr kostengünstig, da es sehr schnell optimal sterilisiert (durch das Autoklavieren) und häufig eingesetzt werden kann.

Für die hermetische Abdichtung ist bei einer besonderen Ausgestaltung des erfindungsgemäßen Endoskops das distale Ende des Schafts mit einem Deckglas hermetisch abgedichtet (das Deckglas wird bevorzugt mit dem Schaft verlötet), wobei das Objektiv und die zumindest eine Leuchtdiode bevorzugt vom Deckglas beabstandet sind. Dadurch kann sichergestellt werden, daß in dem Innenraum des Schafts, in dem die Bildaufnahmeeinheit integriert ist, kein Wasserdampf während des Autoklavierens eindringen kann.

Insbesondere kann bei dem erfindungsgemäßen Endoskop die zumindest eine Leuchtdiode mit dem Schaft verbunden und von diesem thermisch isoliert sein. Dies ist besonders vorteilhaft für den Fall, daß die zumindest eine Leuchtdiode gemäß ihrer Spezifikation nur Temperaturen ausgesetzt werden kann, die geringer als 130°C sind. In diesem Fall wird die thermische Isolierung derart gewählt, daß während des Autoklavierens die Temperatur der Leuchtdiode nicht über die spezifizierte Maximaltemperatur steigt, obwohl der Schaft auf über 130°C erhitzt wird. Diese thermische Isolierung kann beispielsweise dadurch erreicht werden, daß ein Material zwischen der zumindest einen Leuchtdiode und dem Schaft vorgesehen ist, dessen Wärmeleitfähigkeit so gering ist, daß während des Autoklavierens die Maximaltemperatur für die Leuchtdiode eben nicht erreicht wird.

Natürlich kann auch die Bildaufnahmeeinheit oder Teile davon, sofern dies erforderlich ist, thermisch gegenüber dem Schaft isoliert sein.

Bei einer bevorzugten Ausgestaltung des erfindungsgemäßen Endoskops ist im Endoskop und bevorzugt im Handgriff des Endoskops eine Bilddatenverarbeitungseinheit angeordnet, der die Signale des Bildsensors zugeführt werden. Damit ist es möglich, gleich innerhalb des Endoskops die Signale so aufzubereiten, daß die gewünschten Bilddaten erzeugt werden. Insbesondere können gleich gewünschte Manipulationen der Bilddaten durchgeführt werden, wie z.B. spezielle Filterungen, Drehungen der Bildlage, usw.

Ferner kann im Endoskop, bevorzugt im Handgriff des Endoskops, eine Funkeinheit angeordnet sein, die die Signale des Bildsensors und/oder die Bilddaten der Bilddatenverarbeitungseinheit drahtlos zu einer vom Endoskop getrennten und beabstandeten Empfängereinheit überträgt. Damit ist es möglich, ein elektronisches Endoskop zur Verfügung zu stellen, das keinerlei Kabelzuführungen mehr benötigt (weder für die Stromversorgung noch für die Bilddatenübertragung), so daß jede Behinderung durch solche Kabel bei der Handhabung des Endoskops sicher ausgeschlossen werden kann.

Die Signale des Bildsensors und/oder die Bilddaten können als digitale oder analoge Signale mittels der Funkeinheit übertragen werden. In diesem Fall enthält die Funkeinheit noch, falls nötig, einen Analog-Digital-Wandler oder einen Digital-Analog-Wandler.

Des weiteren kann bei dem erfindungsgemäßen Endoskop am proximalen Ende eine mit dem Endoskop verbundene Bildanzeigvorrichtung vorgesehen sein, auf der die mittels der Bildaufnahmeeinheit aufgenommenen Bilder darstellbar sind. Die Bildanzeigevorrichtung kann als elektronische Anzeige (z. B. ein Flüssigkeitsstallanzeige wie bei Videokameras) ausgebildet sein, so daß Endoskop quasi ein elektronisches Augenstück aufweist. Die elektronische Anzeige kann beispielsweise als LCoS-Anzeige (Liquid Crystal on Silicon - Flüssigkristall auf Silizium-Anzeige) oder auch als LEP-S-Anzeige (Light emitting Polymer on Silicon - lichtemittierendes Polymer auf Silizium) ausgebildet sein. In diesem Fall ist es bevorzugt, daß die im Endoskop vorgesehene Stromversorgungseinheit auch für die elektronische Anzeige als Stromversorgungsquelle dient.

Ferner kann die zumindest eine Leuchtdiode am distalen Ende des Schafts angeordnet sein. Dies ist dahingehend besonders vorteilhaft, daß keine Lichtleiter notwendig sind, da die Leuchtdiode direkt im Bereich der Bildaufnahmeeinheit vorgesehen ist.

Insbesondere sind mehrere Leuchtdioden vorgesehen, die, in einer Ansicht auf das Objektiv gesehen (also entlang der optischen Achse des Objektivs), das Objektiv umgeben. Damit kann eine sehr gleichmäßige Beleuchtung des aufzunehmenden Objekts erreicht werden.

Wenn mehrere Leuchtdioden vorgesehen sind, können beim gepulsten Betrieb der Leuchtdioden die Leuchtdioden in Gruppen oder einzeln abwechselnd pulsierend betrieben werden. Auch ist es möglich, die Helligkeit der Beleuchtung über die Anzahl der gleichzeitig eingeschalteten Leuchtdioden einzustellen.

Die zumindest eine Leuchtdiode ist bevorzugt eine weiße Leuchtdiode, also eine Leuchtdiode, die weißes Licht abstrahlt. Natürlich kann auch eine Leuchtdiode verwendet werden, die farbiges Licht abstrahlt. Ferner ist es möglich, verschieden farbiges Licht abstrahlende Lichtdioden vorzusehen, deren Licht insgesamt eine vorbestimmte Farbe oder weißes Licht ergibt.

Das Endoskop bzw. der Schaft kann flexibel, semi-flexibel oder starr ausgeführt sein.

Die Erfindung wird nachfolgend beispielshalber anhand eines Ausführungsbeispieles mit Bezug auf die beigefügten Zeichnungen noch näher erläutert. Von den Figuren zeigen:
Fig.1 eine Seitenansicht der Ausführungsform des elektronischen Endoskops, und
Fig.2 eine Ansicht des distalen Endes des Schafts des in Fig.1 gezeigten Endoskops.

Das in den Figuren dargestellte autoklavierbare Endoskop ist insbesondere für den Einsatz im medizinischen Bereich vorgesehen und umfaßt einen Schaft 1, der ein äußeres Rohr 2 und ein dazu konzentrisch angeordnetes inneres Rohr 3 aufweist. Das proximale Ende des Schafts (und somit der beiden Rohre 2 und 3) ist hermetisch dicht mit einem Handgriff 4 verbunden, während im inneren Rohr 3 des Schaftes 1 am distalen Ende des Schafts eine Bildaufnahmeeinheit 5 angeordnet ist, die ein Objektiv 6 und einen dem Objektiv 6 nachgeordneten Bildsensor 7 umfaßt. Die Bildaufnahmeeinheit 5 ist bevorzugt fest mit dem Schaft 1 verbunden, so daß die Bildaufnahmeeinheit 5 ortsfest im Schaff 1 angeordnet ist. Natürlich kann das Objektiv aber z. B. als Zoom-Objektiv ausgebildet sein. Der Bildsensor 7 ist bevorzugt ein CCD- oder CMOS-Bildsensor.

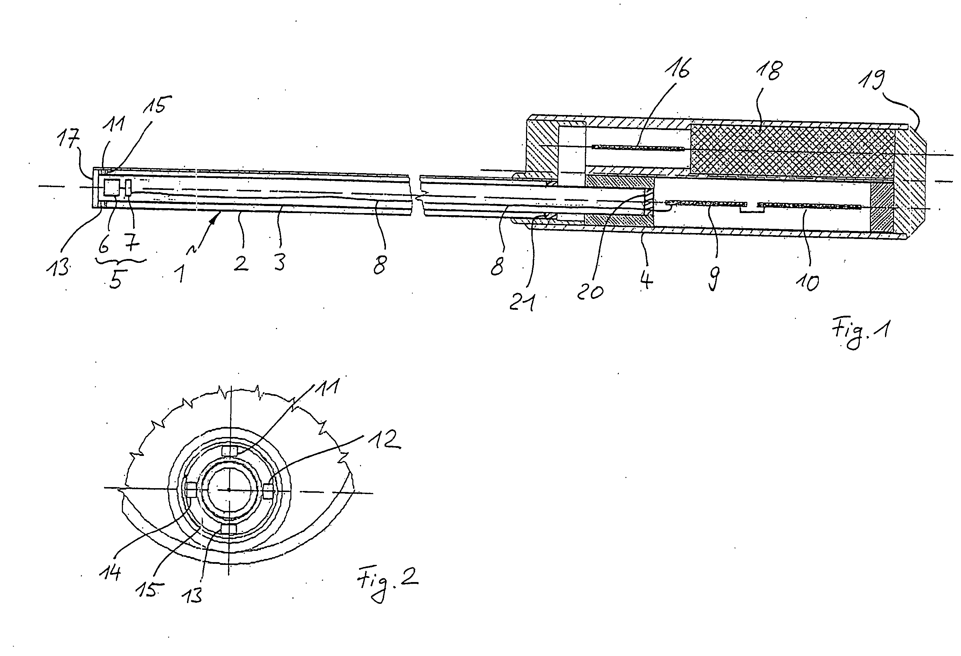

Über eine elektronische Kabelverbindung 8, die in Fig.1 schematisch eingezeichnet ist, ist der Bildsensor 7 mit einer im Handgriff 4 angeordneten Bilddatenverarbeitungseinheit 9 verbunden. Die Bilddatenverarbeitungseinheit 9 verarbeitet die Signale des Bildsensors 7 zu Bilddaten und überträgt diese über ein Funkmodul 10 zu einem nicht gezeigten Empfänger, der vom Endoskop beabstandet und getrennt angeordnet ist. Bei dem Empfänger kann es sich um einen herkömmlichen Computer mit Bildschirm und einem entsprechenden Funkempfangsmodul handeln, wobei der Computer die übertragenen Bildsignale als Bild auf den Bildschirm darstellt.

Ferner sind am distalen Ende des Schafts zwischen dem äußeren und inneren Rohr 2, 3 vier Leuchtdioden 11, 12, 13 und 14 angeordnet. In dem hier beschriebenen Ausführungsbeispiel sitzen die Leuchtdioden 11 bis 14 auf einem ringförmigen Träger 15, der genau zwischen das äußere und innere Rohr 2,3 paßt. Die Leuchtdioden 11 bis 14 sind über eine (nicht gezeigte) Kabelverbindung, die zwischen dem äußeren und inneren Rohr 2,3 verläuft, mit einer im Handgriff 4 vorgesehenen Steuereinheit 16 verbunden. Bei den Leuchtdioden 11 bis 14 handelt es sich bevorzugt um weiße Leuchtdioden. Natürlich ist es auch möglich, je nach Anwendungsfall, unterschiedlich farbige Leuchtdioden 11 bis 14 vorzusehen.

Das distale Ende des Schafts 1 ist durch ein Deckglas 17 hermetisch dicht abgeschlossen.

Im Handgriff 4, der beispielsweise auch als Pistolengriff ausgebildet sein kann, ist ferner noch eine interne Stromversorgung (Stromversorgungseinheit) 18 angeordent. Die Stromversorgungseinheit 18, bei der es sich in dem hier beschriebenen Beispiel um einen induktiv aufladbaren Akkumulator handelt, dient zur Stromversorgung des Bildsensors 7 sowie der Steuereinheit 16, der Bilddatenverarbeitungseinheit 9 und des Funkmoduls 10. Das proximale Ende des Handgriffs 4 ist mit einem Deckel 19 hermetisch abgeschlossen. Damit ist das gesamte Endoskop autoklavierbar.

Alternativ ist es auch möglich, daß der Endoskopschaft 1 lösbar mit dem Handgriff 4 verbunden ist und eine Abdichtung 20 für das innere Rohr und eine Abdichtung 21 für den Zwischenraum zwischen dem inneren und äußeren Rohr 2,3 am proximalen Ende des Schaftes 1 aufweist, so daß der gesamte Schaft 1 autoklavierbar ist. In diesem Fall müssen natürlich die elektrischen Verbindung zur Bilddatenverarbeitungseinheit 8 und zur Steuereinheit 16 lösbar sein.

Die Steuereinheit 16 dient dazu, die Leuchtdioden 11 bis 14 gepulst zu betreiben, wobei der gepulste Betrieb mit der Bildaufnahmewiederholungsrate des Bildsensors 7 synchronisiert ist, so daß das aufzunehmende Objekt immer nur dann beleuchtet wird, wenn auch tatsächlich Bild mittels der Bildaufnahmeeinrichtung 5 aufgenommen wird.

Natürlich kann die Stromversorgungseinheit 18 auch auswechselbar vorgesehen werden. In diesem Fall kann die Stromversorgungseinheit 18 eine Batterie, einen Akkumulator oder auch eine Brennstoffzelle umfassen.

Ferner ist es auch möglich die Kabelverbindung 8 durch eine drahtlose Verbindung zu ersetzen. Das gleiche gilt für die Verbindung zwischen der Steuereinheit 16 einerseits und den Leuchtdioden 11 bis 14 andererseits.

Auch ist es möglich, die Verbindung zwischen der Stromversorgungseinheit 18 einerseits und den Leuchtdioden 11 bis 14 und der Bildaufnahmeeinheit 5 drahtlos zu gestalten, z. B. durch ein induktive Kopplung zu ersetzen.

## Patentansprüche

1. Elektronisches Endoskop mit einem Schaft (1), einer Bildaufnahmeeinheit (5), die ein Objektiv (6) sowie einen diesem nachgeordneten Bildsensor (7) zur Aufnahme eines Objekts aufweist und die am distalen Ende des Schafts (1) fest mit diesem verbunden ist, einem Handgriff (4), der mit dem proximalen Ende des Schafts (1) verbunden ist, sowie mit einer mit dem Schaft (1) verbundenen Beleuchtungseinrichtung (11, 12, 13,14) zum Beleuchten des aufzunehmenden Objektes, **dadurch gekennzeichnet, daß** die Beleuchtungseinrichtung (11, 12, 13,14) zumindest eine Leuchtdiode (11, 12, 13, 14) aufweist.

2. Endoskop nach Anspruch 1, **dadurch gekennzeichnet, daß** im Endoskop, insbesondere im Handgriff (4), eine Steuereinheit (16) angeordnet ist, mit der die zumindest eine Leuchtdiode (11, 12, 13, 14) gepulst betreibbar ist, wobei bevorzugt der gepulste Betrieb der zumindest einen Leuchtdiode (11, 12, 13, 14) mit der Bildaufnahmerate des Bildsensors (7) synchronisiert ist.

3. Endoskop nach einem der obigen Ansprüche, **dadurch gekennzeichnet, daß** eine Stromversorgungseinheit (18) für die zumindest eine Leuchtdiode (11, 12, 13, 14) und die Bildaufnahmeeinheit (7) im Endoskop, insbesondere im Handgriff (4), integriert ist.

4. Endoskop nach einem der obigen Ansprüche, **dadurch gekennzeichnet, daß** das Endoskop derart hermetisch abgedichtet ist, daß des autoklavierbar ist, oder daß der Schaft (1) derart hermetisch abgedichtet ist, daß der Schaft (1) autoklavierbar ist.

5. Endoskop nach Anspruch 4, **dadurch gekennzeichnet, daß** das distale Ende des Schafts (1) mittels einem Deckglas (17) hermetisch abgedichtet ist und das Objektiv (6) und die zumindest eine Leuchtdiode (11, 12, 13, 14) vom Deckglas (17) beabstandet sind.

6. Endoskop nach einem der obigen Ansprüche, **dadurch gekennzeichnet, daß** die zumindest eine Leuchtdiode (11, 12, 13, 14) mit dem Schaft (1) verbunden und von diesem thermisch isoliert ist.

7. Endoskop nach einem der obigen Ansprüche, **dadurch gekennzeichnet, daß** im Endoskop, bevorzugt im Handgriff (4), eine Bilddatenverarbeitungseinheit angeordnet ist, der Signale des Bildsensors (7) zugeführt werden und/oder daß eine Funkeinheit (10), bevorzugt im Handgriff (4), angeordnet ist, die Signale des Bildsensors (7) oder Bilddaten der Bilddatenverarbeitungeinheit (9) drahtlos zu einer vom Endoskop beabstandeten und getrennten Empfängereinheit überträgt.

8. Endoskop nach einem der obigen Ansprüche, **dadurch gekennzeichnet, daß** am proximalen Ende des Endoskops eine Bildanzeigevorrichtung vorgesehen ist, auf der die aufgenommenen Bilder darstellbar sind, und/oder daß die zumindest eine Leuchtdiode (11, 12, 13, 14) eine weiße Leuchtdiode ist.

9. Endoskop nach einem der obigen Ansprüche, **dadurch gekennzeichnet, daß** die zumindest eine Leuchtdiode (11, 12, 13, 14) am distalen Ende des Schafts (1) angeordnet ist, wobei bevorzugt mehrere Leuchtdioden, (11, 12, 13, 14) derart am distalen Ende des Schafts (1) angeordnet sind, daß sie, in einer Ansicht auf das Objektiv (6) gesehen, das Objektiv (6) umgeben.

10. Endoskop nach einem der obigen Ansprüche, **dadurch gekennzeichnet, daß** der Schaft (1) flexibel oder starr ausgebildet ist.
